# EUROPEAN PATENT SPECIFICATION

(11) **EP 3 142 615 B1**
(45) Date of publication and mention of the grant of the patent: **23.02.2022**
(21) Application number: 15721815.7
(22) Date of filing: 08.05.2015
(51) Int. Cl.: A61F 13/00, A61F 13/02, A61F 13/15

(54) **WOUND DRESSING**
WUNDAUFLAGE
PANSEMENT

(30) Priority: 14.05.2014 GB 201408553; 14.05.2014 GB 201408547; 14.05.2014 GB 201408549; 20.10.2014 GB 201418565
(43) Date of publication of application: 22.03.2017
(73) Proprietor: Brightwake Limited, Nottingham, Nottinghamshire NG17 7JZ (GB)
(72) Inventor: COTTON, Stephen, Nottingham Nottinghamshire NG15 8EQ (GB)
(74) Representative: Adamson Jones
(86) International application number: PCT/GB2015/051365
(87) International publication number: WO 2015/173547

(56) References cited:
- EP-A1- 2 545 943
- WO-A1-03/026544
- WO-A1-2010/061228
- WO-A1-2011/001181
- DE-A1-102006 017 194
- GB-A- 2 496 310
- US-A- 4 541 426
- US-A1- 2004 054 313
- US-A1- 2012 215 193
- US-A1- 2014 066 868

## Description

This invention relates to a wound dressing, and in particular to a wound dressing comprising honey that, in use, is released into the wound to aid in wound healing or to provide some other therapeutic benefit.

Different types of wound dressing are required to meet different clinical needs. However, there are several desirable characteristics that are common to all wound dressings. These include the ability to absorb exudate from a wound. In addition, infrequent dressing changes are preferable as changing a dressing can aggravate a wound, as well as causing pain and/or discomfort for the patient. Pain-free removal and the ability to remove a dressing without trauma to the wound and the surrounding skin are two additional important characteristics. In order to prevent pain and trauma, the facing layer of a wound dressing needs to maintain a moist layer over the wound to prevent adherence to the drying wound. It is also desirable for a wound dressing to include some form of adhesive layer to maintain it in position, but repeated removal and replacement of such dressings can damage the peri-wound skin, ie the skin adjacent to the wound. The term "atraumatic dressing" is used in relation to products that, upon removal, do not cause trauma either to newly formed tissue or to the peri-wound skin. Dressings that do not adhere to the surface of a wound are (notwithstanding that they adhere to the healthy peri-wound skin) termed "non-adherent" or "low-adherent".

In addition, dressings should be able to absorb exudate from a wound, while retaining sufficient structure that they can be easily removed from the wound after use. If a wound dressing cannot be cleanly removed from a wound then a patient will suffer additional trauma. In addition, if fragments of the dressing are left in the wound, healing may be inhibited. In addition to the need to retain structural integrity, it is important for many applications that a wound dressing is able to absorb a significant amount of liquid. During the healing process, wounds produce exudate. This is absorbed by the dressing in order to keep the wound clean and promote healing. A determining factor in how regularly a dressing needs to be changed is how quickly the dressing becomes saturated with exudate. Infrequent dressing changes are preferable as changing a dressing can aggravate a wound, as well as causing pain and/or discomfort for the patient. It is essential for efficient wound healing that the area of the wound be kept moist but the surrounding skin be kept dry to avoid maceration.

Infection of wounds during the healing process is a major concern in healthcare and, as well as being a source of pain and distress to the patient, can be very expensive. Infection of a wound can greatly increase the length of a patient's stay in hospital. However, pre-emptive prescribing of antibiotics is not currently recommended due to the rising number of resistant strains of bacteria. It is therefore known to incorporate antimicrobial agents or other therapeutic benefit agents into a wound dressing.

One benefit agent that has been included in wound dressings is honey. Healing properties have long been associated with honey. Honey is a mixture of sugars, water and other active ingredients such as vitamin C, catalase and chrysin, that are thought to act as anti-oxidants. As such, honey has much greater activity than sugar alone.

In particular, certain honeys such as manuka honey, produced from the nectar of the plant species *Leptospermum scoparium,* have been shown to have high antibacterial, non-peroxide activity which inhibits the growth of various species of bacteria. As such, honey can support wound healing through its anti-inflammatory action, its natural anti-bacterial properties, its debriding action and its stimulatory effect on granulation and epithelialisation. In fact, honey has been shown to have considerable wound- and ulcer-healing capacity and strong antimicrobial capacity even in moist healing environments.

Dressings are known in which honey is simply impregnated into a fibrous mat. The mat may be a gauze-like material, or may be formed of a gelling fibre such as alginate. Such dressings have been commercially available for many years, for instance under the trade names ACTIVON and ALGIVON (Brightwake Ltd, Kirkbyin Ashfield, United Kingdom, www.advancis.co.uk). Whilst such dressings are effective, release of honey from them may not occur in the manner that is desired. In particular, the honey may be washed out of the dressing and away from the wound by wound exudate more quickly than is desired. Also, honey is somewhat sticky and as a result the dressings are not as easy to handle for clinical staff as may be desired.

Another approach is described in WO01/41776, which discloses putty-like compositions comprising honey and a gelling agent. Such compositions can be formed into sheets or moulded to complex shapes, eg for insertion into cavity wounds. Again, such dressings may be of benefit in certain situations, but they also suffer from disadvantages. The absorptive capacity of such dressings is very limited, and the release of honey may be lower than is desired, as a result of the phenomenon known as gel-blocking, in which wound exudate combines with gelling agent at the surface of the dressing to form a layer of gel that prevents the release of honey from within the dressing.

Another form of wound dressing is disclosed in WO2011/001181. This dressing comprises a dissolvable film that contains a small proportion - typically around 4% w/w - of propolis, another known antimicrobial agent that is produced by bees. The application of such a film directly to a wound to aid wound healing is described. The possibility of incorporating such a film into a composite wound dressing is also mentioned, as is the use of such material to form a sachet to hold superabsorbent material in a wound dressing, but no example of such a dressing construction is given.

It has now surprisingly been found that antimicrobial and other therapeutic benefit agents, notably honey, may be incorporated into solid dissolvable films at high concentrations and that such a film may be laminated to an absorbent body to provide a wound dressing that overcomes or substantially mitigates some or all of the above-mentioned and/or other disadvantages of the prior art.

Thus, according to a first aspect of the invention, there is provided a wound dressing comprising an absorbent body that is laminated to, or is in intimate contact with, a perforated film comprising honey, wherein the film is formed with a regular array of perforations and, in use, is exposed to a wound to which the dressing is applied, such that exudate from the wound passes through perforations in the film and is absorbed by the absorbent body, and wherein the film dissolves upon exposure to wound exudate, thereby releasing the honey.

The wound dressing according to the invention is beneficial in that the absorbent body provides for good absorption of wound exudate, while the perforated film slowly dissolves as a result of contact with the wound exudate. Dissolution of the film releases the honey into the vicinity of the wound. This leads to a high and prolonged concentration of honey at the wound site. The perforations nonetheless permit the flow of wound exudate into the absorbent body, without the occurrence of gel-blocking or similar phenomena. Prior to use, the perforated film is dry, which may make the dressing easier to handle and apply. In addition, the film may be sufficiently thin that it does not significantly reduce the flexibility and conformability of the dressing.

### Perforated film

The perforated film preferably comprises a carrier material that is flexible at ambient temperature, and which dissolves at an appropriate rate in use, upon exposure to wound exudate. The carrier is preferably capable of forming conformable and flexible sheets that are sufficiently robust to maintain their integrity during normal use and handling. As the perforated film is placed in direct contact with a wound, the carrier is most preferably free from toxic or allergenic substances. The carrier may be formed of a single substance, or may be a composite. The carrier is preferably a polymeric material.

The film may comprise up to 60% w/w carrier, but more commonly the proportion of carrier material is up to 50% w/w, for instance from about 10% to about 60%, or from about 20% to about 50%, or from about 30% to about 50% w/w.

Suitable materials for use as, or in, the carrier include many natural or synthetic polymers. The carrier materials should be such that, in use and once applied to a wound, the carrier dissolves or otherwise breaks down over time, thereby releasing the honey into the wound environment.

In many embodiments, the carrier comprises a polymer with film-forming properties. A variety of suitable film-forming polymers may be used, provided that they are suitable for medical applications, for instance they are non-toxic and biocompatible.

Most commonly, the carrier comprises just one polymer, which may be a film-forming polymer. Alternatively, the carrier may comprise a mixture of materials, one or more of which may be a film-forming polymer.

Most preferred carriers are soluble upon exposure to wound fluid. Use of such carriers facilitates the release of the honey into the wound space when the antimicrobial material is in place. The most preferred substance for use as a carrier is polyvinyl alcohol (PVA).

Other synthetic polymers that may be suitable for use in the antimicrobial material are biodegradable polyesters. Specific examples of such polymers are polylactic acid and polyglycolic acid, and copolymers and blends thereof. Other examples include polycaprolactones and polyhydroxyalkanoates, such as polyhydroxybutyrate, polyhydroxyvalerate and polyhydroxyhexanoate.

Further film-forming polymers that may be suitable for use in the antimicrobial material are polysaccharides, and in particular basic polysaccharides.

One such film-forming basic polysaccharide is chitosan, the (at least substantially) N-deacetylated derivative of chitin. Chitin is a naturally abundant mucopolysaccharide and is the supporting material of crustaceans and insects. Chitin is readily obtainable from crustacean shells discarded in the food industry, and the preparation of chitosan from chitin is well known and documented.

Derivatives of chitosan may also be used. Chitosan may offer further benefits when used as the carrier material. In particular, chitosan is known to swell when hydrated and so may absorb wound exudate. In addition, chitosan may itself exhibit useful antibacterial and haemostatic properties. Chitosan is also believed to be susceptible to attack by lysozyme, and this may be useful in providing a ready mechanism for biodegradation of the carrier material.

Examples of other synthetic polymers that may be suitable are aminated polymers such as aminated PEGs (including those sold under the trade name JEFFAMINE) and polyallylamines.

The carrier material is chosen such that it dissolves or otherwise breaks down over time, so that the honey is released from it. The material may be such that the film, at least in that part which is in contact with wound fluid, loses its integrity over a timescale of several minutes, for example up to 10, 30 or 60 minutes, eg 1 to 10 minutes or 10 to 60 minutes, or several hours, for example up to 6, 12 or 24 hours, eg 1 to 6 hours or 6 to 24 hours, or several days, for example up to 1 day or 2 days.

One or more additives may be included to improve the physical properties of the film. For instance, the incorporation of an additive may improve the flexibility and pliability of the film.

Typically, the perforated film has a thickness of from 20µm to 5mm, but more commonly, the thickness of the film is from 100µm to 3mm, more typically 200µm to 2mm, eg about 0.5mm or about 1mm.

The perforated film may be manufactured in various ways. A currently preferred method of manufacture, which constitutes a further aspect of the invention, comprises the steps of:
a) producing a fluid precursor composition comprising a carrier material, honey, and a liquid medium; and
b) casting the precursor composition into a sheet;
c) causing or allowing the precursor composition to at least partially solidify; and
d) forming perforations in the at least partially solidified film.

In the currently preferred manufacturing method, the precursor composition most preferably contains about 20% to about 80% w/w of liquid medium. The liquid medium is employed in the precursor composition to aid in the mixing of the components and to enable the composition to be cast into the desired form prior to it being caused or allowed to solidify. The liquid medium is preferably non-toxic and non-allergenic, to prevent an adverse reaction when the film comes into contact with a wound. In the currently preferred manufacturing method, PVA is the preferred carrier, and water is the preferred liquid medium.

The precursor composition may be prepared by dispersing the carrier material (eg PVA) and the honey in the liquid medium. The carrier material is preferably added to the liquid medium in finely divided or powder form and the liquid medium may be heated and/or agitated to aid dispersion.

In one embodiment, the precursor composition may be spread onto a substrate to form a layer of uniform thickness. The substrate may be a film, eg of paper or plastics material, that functions as a release carrier from which the film is peeled or otherwise separated prior to incorporation into a wound dressing of the invention.

Once the precursor composition has been cast into the desired form, it is caused or allowed to solidify. This process involves subjecting the precursor composition to different conditions depending on the nature of the carrier material. In particular, this may involve heating the precursor composition for a period of time in order to evaporate some or all of the liquid medium and/or cause the carrier material to set. For instance, the precursor composition may be heated in an oven at a temperature above ambient temperature but generally below about 100°C, eg between 40° and 70°C for a period of between 5 minutes and 1 hour, eg 15-30 minutes. Heating of the precursor composition generally results in the loss by evaporation of any volatile components. The liquid medium will generally be at least partially aqueous, and heating will also result in the loss of much of the water that is present. Typically, the film has a water content of less than 20% w/w, more commonly less than 10%, eg 1 to 20% or 1 to 10% w/w, for instance about 5% w/w.

The heating process may be carried out batchwise, but is preferably carried out as a continuous process, eg by transporting the precursor composition through an oven on a conveyor.

The method may involve the use of a manufacturing line that is able to continuously form the film. In one such method, a release carrier is fed onto a conveyor, preferably comprising one or more looped belts, which transports it along the manufacturing line. Suction may be applied from beneath the belts to hold the release carrier flat against the surface of the conveyor.

The precursor composition is preferably applied to the release carrier in a uniform layer. This may be carried out by applying the composition from the edge of a suitably formed blade that is positioned close to the release carrier passing beneath it on the conveyor. Following application of the layer of precursor composition, the product may be passed through an elongated oven.

Once the product has at least partially solidified, perforations are introduced into it. Perforation may take place with the release liner in place, or the release liner may first be removed. In general, "partially solidified" means in the context of the invention solidified sufficiently for perforations to be formed that retain their integrity during subsequent further solidification of the film.

The primary purpose of the perforations is to permit fluid, notably wound exudate and the like, to pass through the film into the absorbent body. The perforations may, however, also provide for ease of removal of the dressing, improved flexibility and conformity, and skin breathability.

The film is formed with a regular array of perforations. Typically, such perforations are circular and have a diameter of from 50µm to 10mm, more commonly from 1mm to 5mm. Other shapes of perforation may alternatively be used, eg square perforations or elongated slits.

The perforations may be produced by mechanical cutting, eg using a rotary or reciprocating cutting die.

A currently preferred method of forming the perforations, however, involves the application of high frequency mechanical vibrations in a similar manner to ultrasonic welding technology. This method involves contacting perforating elements with the coated or uncoated film and subjecting the film, at least in the regions contacted with the perforating elements, to high frequency mechanical vibrations.

The application of high frequency mechanical vibrations to the film brings about the generation of localised heat by friction, which leads to softening of the material, thereby facilitating puncturing of the material by the perforating elements.

The high frequency mechanical vibrations are preferably applied to the material using a device of the type commonly used in ultrasonic welding. These devices are typically used to weld thermoplastic or fine metal components by applying high frequency mechanical vibrations to such components as they are held together under pressure. This combination of mechanical vibration and pressure results in the generation of heat by friction, allowing the generation of heat to be localised to the points at which the material is held under pressure. The use of ultrasonics is of particular advantage in the medical industry because it does not introduce potential contaminants into the material. The use of ultrasonics is advantageous compared to the direct application of heat to the material because it is highly controllable and may be switched off instantaneously without any residual effect. Excess or residual heat is undesirable because it may damage the film or cause it to deform. Also, the effects of ultrasonics can be restricted to a very limited part of the material without altering the properties of the surrounding regions.

The film is generally held between the perforating elements and a sonotrode, by which the high frequency vibrations are applied. The perforating elements preferably take the form of a plurality of projections extending from a support, such that the tips of the perforating elements contact the film. The sonotrode may then be applied to the other side of the material so as to hold the film under pressure between the sonotrode and the support, compressing the film between the sonotrode surface and the projections at the points at which it is in contact with the tips of those projections. The generation of heat by friction is thereby localised to the points of the film that are in contact with the tips of the perforating elements. The perforating elements may then pass through the film at these points, producing perforations. The perforating elements thereby serve to compress the film against the sonotrode at the desired points, localising the generation of heat to the points at which they contact the film, followed by perforation of the film at those points.

The perforating elements most preferably pierce the film as soon as possible following contact with the sonotrode. It is therefore desirable to apply a force to the film to facilitate passage of the perforating elements through it. This may be done by applying suction, by holding the film under tension against the perforating elements, or by applying a mechanical force directly to the film.

The support from which the perforating elements extend preferably takes the form of a roller with the perforating elements extending from its circumferential surface. Such a roller will typically have a diameter of between 5cm and 50cm, more commonly between 10cm and 30cm. The film may be fed on and off the roller and make contact with the sonotrode continuously, improving throughput. The sonotrode must therefore apply high frequency mechanical vibrations to the material continuously. It is therefore necessary to supply the high frequency mechanical vibrations to the sonotrode using a continuous pulsating generator, rather than an intermittent pulsating generator, both of which are commonly used in the field of ultrasonics.

Generally, operation of the sonotrode for continuous periods will, unless appropriate measures are put in place to maintain the temperature of the sonotrode at a substantially constant level, result in the generation of heat and an increase in the temperature of the sonotrode. This can lead to thermal expansion of the sonotrode, which may reduce the clearance between the sonotrode and the perforating elements. It may therefore be desirable or necessary for the sonotrode to be cooled during operation, eg by the application of a cooling fluid, most commonly chilled air.

The film on which the process is carried out is typically in the form of an elongate strip with a width that generally does not exceed 200mm, although the use of strips with greater widths is possible. However, sonotrodes having a width of greater than about 200mm are less effective at applying high frequency mechanical vibrations to a material. Therefore, in order to perforate strips of film material having widths in excess of 200mm, a number of sonotrodes positioned adjacent to one another may be used.

The film is preferably fed past the perforating elements at a rate of at least 0.1 metres/second and up to 1.0 metres/second. Typically, the film may be fed through the apparatus at a rate of between 0.2 and 0.8 metres/second, or between 0.3 and 0.6 metres/second.

Alternatively, the film may be laminated to an absorbent body prior to forming the perforations. In this case the film may be manufactured as set out above up to the point at which the film would be perforated. The film is then laminated to the absorbent body prior to being perforated. Preferably the film is laminated to the absorbent body by the application of heat and pressure. The perforations may then subsequently be formed through both the film and absorbent body of the resulting laminate.

Perforating both the film and the absorbent body may result in a dressing which is more breathable and also allows for easier passage of wound exudate away from the wound.

The perforations in the laminate may be formed by similar processes to those discussed above in relation to the perforation of the film itself, ie by mechanical cutting or by application of high frequency mechanical vibrations.

Preferably the perforations are formed by application of high frequency mechanical vibrations. Forming the perforations in this way compresses the film and the absorbent body between the sonotrode and the support. The resulting laminate may have a quilted appearance.

### Benefit agents

The perforated film comprises honey, as a benefit agent. By "benefit agent" in the context of this invention is meant a material that has a therapeutically beneficial effect at the wound site to which a dressing of the invention is applied. For example, benefit agents may be (without limitation) antimicrobial agents, antiseptic agents, antifungal agents, anti-inflammatory agents, and/or haemostatic agents.

Honey has long been known to be effective in treating wounds, with records of such use dating from at least 2000 years ago. More recently, research has shown honey to have potent antimicrobial, antifungal and anti-inflammatory properties, and to be able to stimulate lymphocytic and phagocytic activity within the body. Further, honey has been demonstrated to assist in the debridement of necrotic tissue, and to stimulate the growth of new tissue. In terms of its antibacterial activity, honey has been reported to have an antibacterial effect on more than sixty species of bacteria, including aerobes and anaerobes, and both Gram-positive and Gram-negative bacteria. In particular, honey has been shown to be effective against antibiotic resistant strains of bacteria including MRSA.

Without wishing to be bound by theory, it is believed that the antibacterial activity of honey is partly due to the release of low levels of hydrogen peroxide, a well known antimicrobial agent. As the production of hydrogen peroxide is stimulated by dilution of the honey (eg by wound exudate), honey has the distinctive property of becoming more active on dilution, rather than less.

Many studies have shown that the maintenance of a moist wound environment aids in wound healing. However, a moist environment also promotes the growth of bacteria, and the prevention of infection is therefore a serious concern. The addition of honey to a wound dressing thus enables the wound to be kept moist whilst inhibiting bacterial growth and reducing the likelihood of infection.

Honey is produced worldwide from many different floral sources, and its antibacterial activity varies with the source of the honey and the processing it has undergone. For example, lotus honey in India is reputed to be good for eye diseases, whereas manuka honey, a monofloral honey produced from the nectar of the manuka bush, is known for its antiseptic properties. The manuka plant is part of the genus *Leptospermum,* and honeys produced from plants of this genus, such as manuka or jellybush honey, are known for their particularly strong antibacterial properties. Preferably, the honey used in the present invention is produced from plants of the genus *Leptospermum.* More preferably, the honey is manuka honey or jellybush honey.

It has been found that in the perforated film incorporating honey, the honey may be incorporated into the film at surprisingly high concentrations. In particular, honey may account for more than 25%, more than 35% or more than 45% w/w of the film, and up to 55%, 65% or 75% w/w of the film. The honey content of the film may therefore be from about 25%, 35% or 45% to about 55%, 65% or 75% w/w, for instance from about 25% to about 75%, from about 35% to about 65%, or from about 45% to about 55% w/w.

Disclosed herein is a solid, dissolvable film comprising a polymeric carrier material and from about 25% to about 75%, from about 35% to about 65%, or from about 45% to about 55% w/w honey. The honey is preferably manuka honey or jellybush honey, and the carrier is preferably polyvinyl alcohol. Such films may be of utility in the treatment of wounds, more generally than in the perforated and laminated form of the first aspect of the invention.

Another antimicrobial agent that may be incorporated into the perforated film is silver. Despite metallic silver being relatively unreactive, ionic silver has been shown to have antimicrobial activity and has been previously used in wound dressings. In use, positively charged silver ions bind to negatively charged sites on proteins and nucleic acids in bacteria. This causes a number of effects, including alteration of the protein structure, rupture of the cell wall and/or cell death. It is believed that silver ions have multiple attack sites, interacting with a number of different functional groups in bacteria, including thiol groups, carboxylates, phosphates, indoles and amines. This makes the development of bacterial resistance to silver unusual.

The incorporation of silver into the film results in the sustained release of low concentrations of silver ions over time. Such a slow release has been shown to stimulate healing and inhibit the growth of micro-organisms. Any suitable method or form of silver known in the art may be used in the present invention. For example, the silver may be in the form of silver sulphadiazine.

Another antimicrobial that may be incorporated into the film is polyhexamethylene biguanide (PHMB; also known as polyaminopropyl biguanide). PHMB is available as a 20% aqueous solution under the trade name COSMOCIL^{®} CQ from Arch Personal Care Products, 70 Tyler Place, South Plainfield, NJ 07080, USA.

Further antimicrobials that may be incorporated into the film include plant essential oils having known antimicrobial activity. For instance, where manuka honey is used, the antimicrobial activity may be enhanced by the additional inclusion in the film of a small proportion of manuka oil.

In general, although antimicrobial agents such as silver, PHMB or an essential oil may be incorporated into the film at high concentrations, relatively low concentrations are found in practice to be sufficient. Thus, such an antimicrobial agent is typically present at a level of between 0.1 and 10% w/w, more typically between 0.1 and 5% w/w, more commonly between 0.1 and 2% w/w.

More than one antimicrobial agent is preferably present in the film, to provide activity against a wide range of micro-organisms. As noted above, honey is used, and is preferably present at high concentration. In addition to honey, it is preferred that at least one, and preferably two or more, further antimicrobial agents are present, selected from PHMB, silver and plant essential oils.

Films comprising manuka honey, PHMB and optionally also manuka oil are particularly preferred. In such films, the concentrations of manuka honey, PHMB and essential oil may be as set out above.

Alternatively, or in addition to, antimicrobial agents, the perforated film may incorporate other benefit agents, which may be selected from antifungal agents, antiviral agents, anti-inflammatory agents, and haemostatic agents.

### Absorbent body

The wound dressing according to the invention comprises an absorbent body, which is laminated to, or is in intimate contact with, the perforated film. Preferably, the film is laminated to the absorbent body.

Any suitable absorbent material may be used in the wound dressing.

The absorbent body may comprise a foam, a gelling material, a superabsorbent material, or a combination of these components.

Where the absorbent body comprises a foam, the foam may be any suitable foam known in the art. Usually, the foam is an open-celled foam. Preferably, the foam is a hydrophilic foam. More preferably, the hydrophilic foam is a polyurethane foam. Most preferably, the foam is an open-celled polyurethane foam. The open cellular structure of the foam allows exudate from a wound to pass through it, as well as to be absorbed by it. The foam typically has a thickness of 0.5mm to 10mm, preferably from 1 mm to 7mm, or from 2mm to 7mm, and the foam most preferably has a thickness of about 2mm, about 3mm, about 4mm or about 5mm.

The absorbent material may alternatively comprise a gelling material. By "gelling material" is meant in relation to the invention a material that is capable of absorbing aqueous fluid, such as wound exudate, and which on absorbing liquid becomes gel-like, moist and slippery. The gelling material is preferably in the form of a non-woven pad or a knitted structure. The gelling material may have an absorbency of at least 2 grams 0.9% saline solution per gram of material, as measured by the free swell absorbency test (ie dispersing a known dry weight of material in the test liquid (saline) for sufficient time for the material to absorb liquid, removing the excess liquid by vacuum filtration, and measuring the increase in weight of the fibre). The absorbency may be considerably higher, eg at least 5g/g, or at least 1 0g/g, or at least 15g/g, or at least 25g/g.

The gelling material may be any suitable gelling material known in the art, including pectin, alginate, materials made from alginate and another polysaccharide, chitosan, hyaluronic acid, other polysaccharide materials or materials derived from gums, or chemically-modified cellulosic materials, eg carboxymethyl cellulose (CMC). The gelling material may be a combination or blend of different gelling materials.

Where the absorbent body is a non-woven pad or knitted structure, it may be formed using fibres or yarns of both gelling and non-gelling material. Typical non-gelling material is cellulosic fibres, eg of tencel or lyocell. The gelling and non-gelling fibres or yarns may be separate, for instance where non-gelling yarns are used to reinforce a structure of gelling yarn, or gelling and non-gelling fibres may be blended in a single yarn. Suitable blended yarns of this type are disclosed in WO2013/064831 .

Currently preferred gelling fibres are alginate fibres and pectin fibres.

In other embodiments of the invention, the absorbent body may comprise a superabsorbent material. "Superabsorbent material" in the context of the present invention means a material that is capable of absorbing many times its own mass of water (eg up to 200, 300, 400, 500 or more times its own mass of water).

Although it should be appreciated that the absorbent body of the present invention may comprise any superabsorbent material, preferred superabsorbent materials are polymeric superabsorbent materials and include alginate, polyacrylate (ie a salt of polyacrylic acid), polyacrylamide copolymers, ethylene maleic anhydride copolymer, carboxymethylcellulose, polyvinylalcohol copolymers, polyethylene oxide and starch-grafted copolymers of polyacrylonitrile.

Many such superabsorbent materials may be used in particulate form. In such cases, the particles may be incorporated into a carrier material, for instance by being encapsulated between two layers of carrier material, eg tissue paper or the like.

Alternatively, the superabsorbent material may be used in a fibrous form, such that fibres of the superabsorbent material form a mat or pad, preferably a non-woven mat or pad.

An alginate superabsorbent may be sodium or calcium alginate. The alginate superabsorbent is preferably in the form of a non-woven mat.

The most preferred superabsorbent material is sodium polyacrylate polymer. Sodium polyacrylate polymer is a solid crystalline material, and is preferably incorporated into a layer in the form of particles encapsulated between two layers of carrier material, such as tissue paper. A specific example of a suitable material is Gelok^{®} 14040S/S manufactured by Gelok International Corporation.

Dressings in which the absorbent body comprises a foam, particularly an open-celled foam, eg an open-celled polyurethane foam, are presently preferred.

The absorbent body may comprise a combination of one or more components. For instance, the absorbent body may comprise two layers of absorbent materials. By way of example, the absorbent body may comprise a foam layer that is laminated to the perforated film, and a superabsorbent layer bonded to the reverse side of the foam layer. In such cases, the foam layer is preferably an open-celled polyurethane foam, and the superabsorbent layer comprises a sodium polyacrylate polymer, for example Gelok^{®} 14040S/S manufactured by Gelok International Corporation. Alternatively, the foam layer is an open-celled polyurethane foam, and the superabsorbent layer is a non-woven mat formed of fibres of a superabsorbent material.

In the first aspect of the invention, the perforated film is preferably laminated to the absorbent body.

Lamination of the perforated film to the absorbent body may be brought about by any suitable means, provided that the perforations in the film are not occluded and passage of wound exudate into the absorbent body is not otherwise significantly inhibited or impaired.

In order to laminate the perforated film to the absorbent body, one or both of the juxtaposed surfaces of those components may be coated with a suitable adhesive, and the two components then pressed together.

Alternatively the two components may be laminated by applying both heat and pressure to the layered components. The two components are typically passed through a pair of heated rollers which compress the layers together. This method of laminating is preferred where the two components are laminated prior to forming the perforations. Rather than perforating the film before laminating it to the absorbent body, both components of the laminate may then be perforated after the laminating process has occurred.

Where the film is perforated before being laminated to the absorbent body, the two components are preferably laminated using a fusible web material. Such materials are typically of synthetic fibres that melt when heated. When the fusible web is placed between the perforated film and the absorbent body and heated, the melting action of the web causes it to fuse the film and absorbent body together.

Fusible web is available in various weights, and in general a lightweight material is preferred, to minimise any risk of the perforations becoming occluded by melted web.

Individual pieces of perforated film and of absorbent material, both having the dimensions necessary for incorporation into a dressing of the invention, may be bonded together using adhesive or fusible web. However, it will be appreciated that more commonly an oversized sheet of perforated film will be bonded to a sheet of absorbent material, and then individual pieces of bonded material will be cut from the sheets, for incorporation into dressings.

An absorbent body comprising a layer of foam bonded to a superabsorbent layer may also be generally used as a wound dressing or as a component in a composite wound dressing. Disclosed herein is an absorbent component for a wound dressing, the component comprising a layer of superabsorbent material bound to a wound-facing layer comprising a foam material.

Superabsorbent materials have been used in wound dressings, particularly in dressings for wounds that produce very high levels of exudate. However, superabsorbent materials are often produced as particulate or fibrous materials and a problem associated with this when such materials are used in wound dressings is that the fibres or particles can transfer into the wound from the dressing. If superabsorbent materials are introduced into the wound in that way, wound healing can be inhibited. For some wounds in particular, for example sinus wounds, it is essential that all of the material is removed when the wound dressing is changed, as cases of giant cell foreign body reaction have been reported. It is therefore important that the superabsorbent material be kept away from the wound surface, but that wound exudate is still allowed to reach the superabsorbent material.

The described absorbent component comprising superabsorbent material for use in a wound dressing has a non-adherent wound-facing surface that allows exudate to reach the superabsorbent material whilst preventing the superabsorbent material from reaching the wound. This component also facilitates the removal of excess exudate from the region of the wound whilst keeping the wound itself moist.

The component is advantageous primarily in that because the superabsorbent material is, in use, not in direct contact with the wound, the risk of fibres or other fragments of superabsorbent material entering the wound is substantially reduced. The wound-facing layer nonetheless permits wound exudate to flow into the superabsorbent material.

The component is also advantageous in that the exudate wets the foam of the wound-facing layer only in the region of the wound and not in the region of the healthy skin surrounding the wound. This avoids maceration of the healthy skin. The exudate travels through the wound-facing layer and is absorbed by the superabsorbent material. The superabsorbent material wicks the exudate away from the wound region. The overall absorbency of the dressing is therefore high but the region of the wound-facing layer that is wetted by the exudate remains local to the wound. Because the exudate is wicked away into the superabsorbent material, the foam layer does not re-wet the wound area.

The foam and superabsorbent material may be any suitable material described above.

### Dressing formats

Wound dressings according to the first aspect of the invention may comprise only the absorbent body and perforated film. When applied to a wound, with the perforated film in contact with the wound, such a simple dressing may be held in place by a secondary dressing or by means of adhesive tapes or the like.

More commonly, however, the wound dressings of the invention are composite dressings comprising additional components. Thus, the dressing typically comprises an adhesive skin contact layer that provides for attachment of the dressing to the peri-wound skin, an absorbent body, and a backing layer that is normally impermeable to liquids but permeable to gas and moisture vapour.

### Skin contact layer

The surface of the dressing or absorbent component that, in use, is applied to the peri-wound skin may carry an adhesive. It is generally preferable that the adhesive that is used is one that is non-adherent and permits the wound dressing to be removed relatively easily and without causing trauma to the wound and surrounding skin. Thus, the adhesive may be, for instance, a hydrocolloid adhesive, a polyurethane adhesive, a hydrogel or, most preferably, a silicone adhesive, particularly a silicone gel.

Silicone adhesives offer numerous advantages. Most preferably, the silicone adhesive is in the form of a silicone gel of the type generally referred to as a "soft silicone".

Soft silicone adhesives are particularly suited for use as skin contact layers in wound dressings. They are soft, tactile and conformable, and exhibit good adhesion to dry skin but low adherence to an underlying wound. Thus, the dressing can be applied to a wound and subsequently removed without causing trauma to the wound. Silicone gels are adhesive but do not leave residue on a surface/substrate when removed.

Silicone gels suitable for use as skin contact materials in the present invention may be carried on a layer of melt-blown non-woven material, eg a sheet of melt-blown polyurethane (MBPU), as described in WO2007/113597.

In such cases, the reverse side of the MBPU may be coated with an adhesive, eg an acrylic adhesive, to affix the silicone gel/MBPU laminate to overlying components of the dressing, eg to the perforated film and/or a backing layer of the form described below.

In use of the dressing of the present invention, it is necessary that the perforated film is exposed to the wound site. Thus, in certain embodiments, the dressing has a border format, in which the adhesive that provides for fixation of the dressing is provided only around the periphery of the dressing, and does not cover the perforated film. Nonetheless, the adhesive skin contact layer may overlap to some extent with the perforated film, provided that the majority of the perforated film is exposed to the wound. In other words, the skin contact layer may be provided with a single, generally central opening, through which the perforated film is exposed to the wound in use.

In other embodiments, the skin contact adhesive may also extend across the perforated film, so that the adhesive layer overlies and contacts the wound itself, rather than just the surrounding healthy skin. In such cases, however, it is necessary that the part of the skin contact layer that does overlie the perforated film is provided with substantial openings, so that a substantial proportion of the perforated film is exposed to the wound. In such cases, it is also particularly important that the skin contact adhesive is one that exhibits low adherence, so that removal of the dressing does not cause undue trauma to the wound.

The skin- or wound-contact layer may be provided with two sets of perforations: the first set allows the transfer of wound exudate through the contact layer and the second set are small enough to avoid significant loss of adherence to the peri-wound skin, so that the dressing has sufficient adherence to remain securely in place during use. The second set of perforations offers an advantage over a dressing in which the peripheral region is unperforated, however, in that the perforations increase the permeability of the dressing to water vapour and to atmospheric oxygen, so reducing the likelihood of maceration of the skin to which the dressing is applied. The perforations may also facilitate removal of the dressing, and provide for improved flexibility and conformability.

In many embodiments, the dressing comprises an absorbent body positioned on the side of the contact layer that is remote from the wound and in fluid communication with the first set of perforations, so that wound exudate may pass through the first set of perforations and be taken up by the absorbent body.

Thus, disclosed is a non-adherent wound dressing comprising an adhesive skinor wound-contact layer provided with perforations, wherein the perforations comprise a first set of perforations of a first dimension in a generally central region of the contact layer that, in use, overlies a wound, and a second set of perforations of a second dimension in a peripheral region of the contact layer that, in use, overlies the peri-wound skin, the first dimension being greater than the second dimension.

The first set of perforations are relatively large (in comparison to the second set) and are located such that they extend across the region of the wound dressing that, in use, is in the proximity of the wound itself. Generally, the first set of perforations are located near the centre of the dressing. Typically, the first set of perforations are circular and have a diameter of from 3mm to 20mm, preferably 3mm to 10mm, or 4mm to 7mm, and most preferably about 5mm.

The second set of perforations are smaller than the first set and are located such that they extend across the border of the dressing on the sections of the dressing that are intended to contact the healthy skin around the wound and not the wound itself. Typically, these perforations are also circular and have a diameter of from 0.5mm to 4mm, preferably from 0.5 to 2mm, and most preferably about 1mm to 2mm.

The presence of different sizes of perforation allows the adhesive dressing to adhere effectively to the healthy skin around the wound whilst also allowing transfer of exudate from the wound through the contact layer, into the absorbent body (if present). If small perforations were present across the full extent of the dressing, transfer of wound exudate may be restricted; if large perforations were present across the full extent of the dressing, adherence of the dressing to the surrounding skin may be reduced.

Other shapes of perforation may alternatively be used, eg square perforations or elongated slits. Some such arrangements of perforations may enhance the extensibility of the dressing in one or more directions, and so improve its conformability to the body.

Thus, the wound dressing of the invention may comprise an absorbent body that is laminated to, or is in intimate contact with, a perforated film comprising honey, wherein the film, in use, is exposed to a wound to which the dressing is applied, such that exudate from the wound passes through perforations in the film and is absorbed by the absorbent body, and wherein the film dissolves upon exposure to wound exudate, thereby releasing the one or more benefit agents, the wound dressing further comprising an adhesive skin- or wound-contact layer provided with perforations, wherein the perforations comprise a first set of perforations of a first dimension in a generally central region of the contact layer that, in use, overlies a wound, and a second set of perforations of a second dimension in a peripheral region of the contact layer that, in use, overlies the peri-wound skin, the first dimension being greater than the second dimension.

The absorbent body of such a wound dressing may be an absorbent component as described above, comprising a layer of superabsorbent material bound to a wound-facing layer comprising a foam material. The layer of superabsorbent material may be any superabsorbent material described in relation to this invention, and may particularly be fibrous, such that fibres of the superabsorbent material form a mat or pad, preferably a non-woven mat or pad.

The skin-contact layer described above may alternatively be incorporated into any wound dressing or composite wound dressing, providing a non-adherent wound contact layer. There is thus disclosed a non-adherent wound dressing comprising an adhesive skin- or wound-contact layer provided with perforations, wherein the perforations comprise a first set of perforations of a first dimension in a generally central region of the contact layer that, in use, overlies a wound, and a second set of perforations of a second dimension in a peripheral region of the contact layer that, in use, overlies the peri-wound skin, the first dimension being greater than the second dimension.

The skin- or wound-contact layer is provided with two sets of perforations: the first set allows the transfer of wound exudate through the contact layer and the second set are small enough to avoid significant loss of adherence to the peri-wound skin, so that the dressing has sufficient adherence to remain securely in place during use. The second set of perforations offers an advantage over a dressing in which the peripheral region is unperforated, however, in that the perforations increase the permeability of the dressing to water vapour and to atmospheric oxygen, so reducing the likelihood of maceration of the skin to which the dressing is applied. The perforations may also facilitate removal of the dressing, and provide for improved flexibility and conformability.

In many embodiments, such a dressing comprises an absorbent body positioned on the side of the contact layer that is remote from the wound and in fluid communication with the first set of perforations, so that wound exudate may pass through the first set of perforations and be taken up by the absorbent body. Where present, the absorbent body is intended to be in close proximity to the wound, and may take any suitable form known in the art, particularly those forms described above in the present application.

### Backing layer

The wound dressing according to the invention may comprise a backing layer, which forms a barrier between the wound and the surrounding atmosphere. Any suitable material known in the art may be used for the backing layer.

The backing layer will generally be impermeable to wound exudate and other liquids, but is preferably permeable to air and moisture vapour. In particular, the backing layer preferably exhibits a relatively high moisture vapour transmission rate (MVTR). The MVTR of the backing layer may be at least 300g/m²/24h, more suitably at least 500g/m²/24h and preferably at least 700g/m²/24h at 37°C and 100% to 10% relative humidity difference.

The backing layer is most preferably a plastics film having the desired characteristics. The backing layer may be a polyurethane film.

The backing layer may be larger in size than the perforated film and absorbent body, such that it extends beyond the edge of the film and absorbent body on one or more sides. Preferably, the backing layer extends beyond the edge of the film and absorbent body on all sides, forming a border around the film and absorbent body, and is bonded to the peripheral region of the skin contact layer.

### Release liner

Where the skin contact layer carries an adhesive, or where the perforated film is tacky (for instance where it contains honey), the dressing will generally be supplied with a releasable liner on its underside. The releasable liner may cover the adhesive portions of the wound dressing prior to use, and be removed from the dressing immediately before application of the dressing to the wound. This reduces the risk of contamination of the wound dressing and facilitates handling of the dressing.

Such releasable liners are commonly used on wound dressings known in the art, and suitable materials which can be employed in the present invention will be familiar to the skilled worker. For example, the releasable liner may be of a suitable plastics sheet or a siliconised paper or the like.

The releasable liner may be a single sheet which covers the underside of the wound dressing, or may be formed of two or more sheets. The releasable liner may further comprise a tab to enable the liner to be easily removed from the dressing before use. In particular, where the releasable liner is formed of two or more parts, the parts may either overlap or abut and extend outwards from the wound dressing, thus providing an easy method for removal of the releasable liner.

Disclosed is provided a method of treating a wound, which method comprises applying to the wound a wound dressing, film or absorbent component according to any of the above described embodiments of the invention.

Embodiments of the invention and disclosure will now be described in greater detail, by way of illustration only, with reference to the following Example and the accompanying drawings, in which
Figure 1 is a plan view of a first embodiment of a wound dressing according to the first aspect of the invention;
Figure 2 is an underside plan view of the wound dressing of Figure 1, with release liners removed;
Figure 3 is a cross-sectional view (not to scale) along the line III-III in Figure 1;
Figure 4 is a schematic representation, not to scale, of apparatus used to produce perforations in a film forming part of the dressing of Figures 1 to 3;
Figure 5 is a plan view of a second embodiment of a wound dressing according to the first aspect of the invention;
Figure 6 is a plan view of a wound dressing as disclosed herein;
Figure 7 is cross-sectional view of the wound dressing of Figure 6, on the line II-II and not to scale; and
Figure 8 is an underside view of the wound dressing of Figures 6 and 7, after removal of a releasable liner.
Figure 9 is a plan view of a further embodiment of a wound dressing according to the invention; and
Figure 10 is cross-sectional view of the wound dressing of Figure 9, on the line X-X and not to scale.
Figure 11 is a plan view of a wound dressing according to an alternative embodiment of the invention; and
Figure 12 is a cross-sectional view of the wound dressing of Figure 11, on the line V-V.

### Example

### Dissolvable antimicrobial film

Ingredients:

| | |
|---|---|
| PVA | 43% w/w |
| Manuka honey | 50% |
| Manuka oil | 1% |
| Cosmocil | 1% |
| Water | 5% |

A precursor composition is produced by dispersing the PVA, Manuka honey, Manuka oil and Cosmocil in appropriate proportions in an excess of hot (100°C) water.

The precursor composition is spread onto a temporary substrate (a plastics or paper sheet) and dried in an oven at 45-65°C for 20 minutes, to the desired final water content of 5% w/w. This process may be carried out by spreading the precursor composition on a continuous band of substrate that is conveyed through an elongate oven.

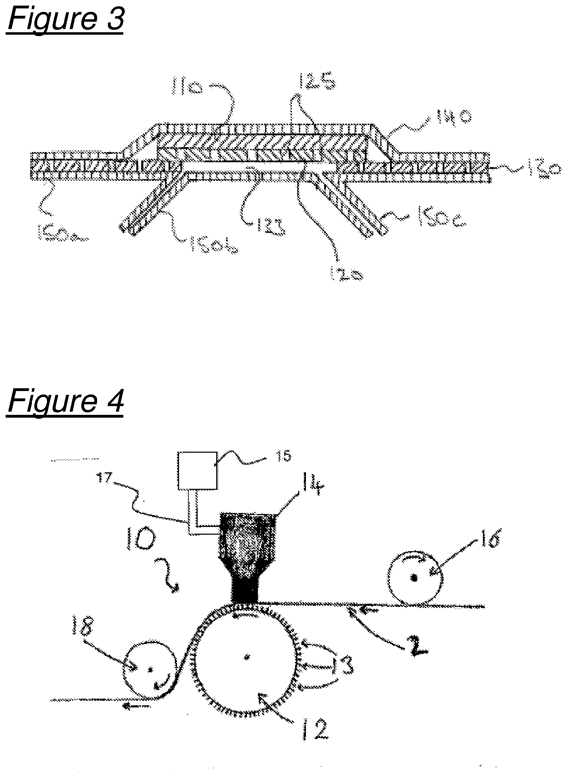

After drying, a regular array of perforations is introduced into the film (still carried on its substrate). Apparatus 10 by which the perforations are introduced into the film is depicted schematically in Figure 4, in which the film/substrate is denoted 2. The perforating apparatus consists of a perforating roller 12 which is a cylindrical barrel having a multitude of flat-tipped, pin-like perforating elements 13 projecting from the circumferential surface, and a sonotrode 14 which, in operation, applies high frequency mechanical vibrations to the film 2. The perforating roller 12 and sonotrode 14 are configured such that when the perforating roller 12 is rotated, the tips of the perforating elements 13 pass close to the surface of the sonotrode 14. The diameter of the perforating roller 12 is approximately 20cm, and the perforating elements 13 have a length of approximately 5mm.

In operation, the film 2 is drawn past a guide roller 16 into the nip between the perforating roller 12 and the sonotrode 14. The points at which the film 2 contacts the tips of the perforating elements 13 of the perforating roller 12 are compressed against the surface of the sonotrode 14. The high frequency mechanical vibrations produced by the sonotrode 14 generate high levels of friction at the points where film 2 is compressed, causing heating of the film 2 at these points. The material of the film 2 melts at those points were such heating occurs, allowing the perforating elements 13 to pass through the film 2, thereby forming perforations.

The perforated film 2 is drawn off the perforating roller 12 via a second guide roller 18. The second guide roller 18 is positioned such that the film 2 remains in contact with the surface of the perforating roller 12 after passing through the nip between the perforating roller 12 and the sonotrode 14. This means that the locally heated material of the film 2 cools somewhat before being drawn off the perforating roller 12 and the perforating elements 13 are withdrawn from the perforations that have been formed, so that the the integrity of the perforations is maintained.

Chilled air from a chiller unit 15 is blown through the sonotrode 14 via a conduit 17. The flow of chilled air is controlled to maintain the temperature of the sonotrode 14 substantially constant, and hence prevent thermal expansion of the sonotrode 14 that would otherwise reduce the clearance between the sonotrode 14 and the tips of the perforating elements 13.

The film 2 is drawn off the perforating roller 12 at a rate of approximately 0.3 metres/second. The perforated film 2 may be taken up on a roller (not shown) for storage or may pass directly to further processing stations.

The perforations are circular, with diameter approximately 1.5mm, and are formed in a regular array across the full extent of the film 2, with separations of approximately 5mm.

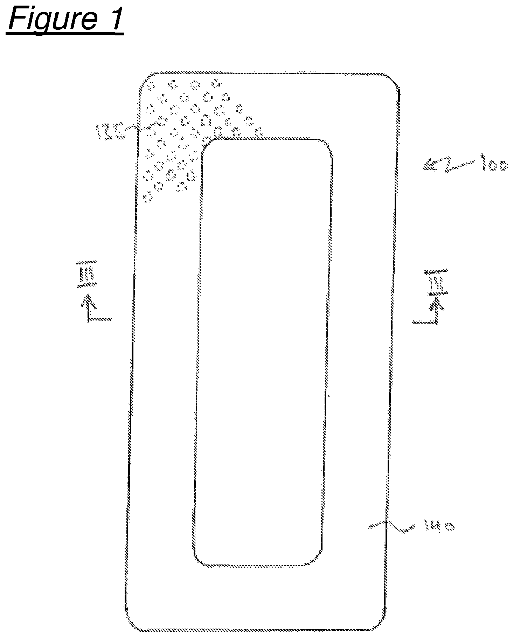

After the film has been formed and perforated, it may be bonded to an absorbent material, eg an open-celled foam, for instance by means of a fusible web interposed between the film and the absorbent material. Appropriately sized pieces of the laminated product may then be cut out and incorporated into a wound dressing, for instance the wound dressing shown in Figures 1 to 3, as described below.

Referring now to the Figures 1 to 3, a wound dressing according to the invention is generally designated 100. The dressing 100 is rectangular in overall shape, with rounded corners. As depicted in the drawings, the dressing 100 has overall dimensions of approximately 25cm × 10cm, but it will be understood that the shape and dimensions of the dressing may vary widely, depending for instance on the size and nature of the wound to be dressed and the part of the body to which the dressing is to be applied.

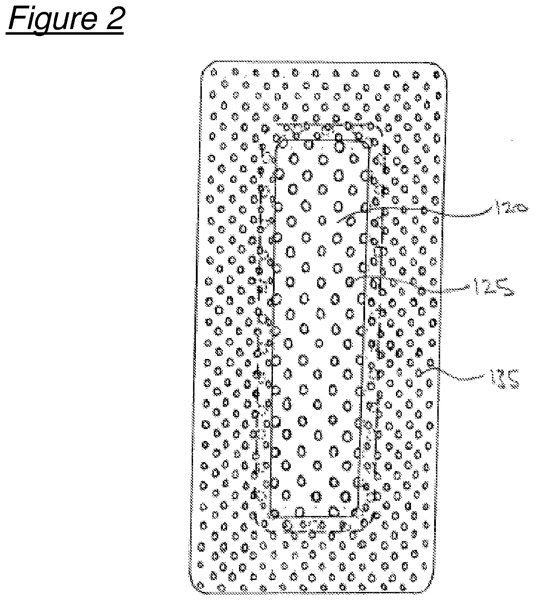

Figure 1 is a plan view of the dressing 100 from above, ie from the non-wound-facing side. The structure of the dressing 100 is more evident from the cross-sectional view of Figure 3 (in which the thickness of the various components is depicted on an exaggerated scale) and the underside plan view (with release liners removed) of Figure 2.

As can be seen most clearly in Figure 3, the dressing 100 comprises an absorbent foam pad 110 that is laminated at its underside (as viewed in Figure 3) to a perforated honey-containing film 120. The film 120 may be a dissolvable antimicrobial film prepared as described in the Example above. The foam pad 110 and film 120 are both substantially rectangular and are of identical length and width. They have been bonded together by means of a fusible web, as described above.

The dressing further comprises a skin contact layer 130 in the form of a sheet of MBPU that is impregnated with and coated on its underside (as viewed in Figure 3) with a silicone gel non-adherent adhesive. The skin contact layer 130 is of greater length and width than the pad 110 and film 120, such that it forms a border around the periphery of the dressing 100.

The skin contact layer 130 has a central opening 133 that is generally rectangular and of slightly smaller dimensions than the film 120 and pad 110, so that the skin contact layer 130 overlaps the edges of the film 120, but the majority of the wound-facing (ie lower, as viewed in Figure 3) surface of the film 120 is exposed. The upper surface of the skin contact layer 130 carries an acrylic adhesive, by means of which the edges of the film 120 are adhered to the periphery of the central opening 133 in the skin contact layer 130.

The dressing is completed by a porous polyurethane backing layer 140 and a three-part release liner 150a-c. The backing layer 140 constitutes the upper surface of the dressing 100. It covers the foam pad 110 and is secured to the border regions of the skin contact layer 130 by means of the acrylic adhesive on the upper surface of the latter. The backing layer 140 may also be adhered to the upper surface of the foam pad 110, but in general it is preferred that the backing layer 140 and foam pad 110 are not so adhered, in order to better provide for swelling of the foam pad 110 during use, as it absorbs wound exudate.

The release liner comprises three overlapping sheets 150a-c of suitable material that are folded to form tabs by which the sheets 150a-c may be pulled away to expose the skin contact layer 140 immediately prior to application of the dressing 100.

As can be seen in Figures 2 and 3, the film 120 is formed with a regular array of perforations 125. For clarity, these are depicted on a somewhat enlarged scale; in reality the perforations 125 have a diameter of approximately 1.5mm and they are separated by approximately 5mm.

As can also be seen in Figures 2 and 3, the border regions of the skin contact layer 130 are also formed with perforations 135. These perforations 135 serve to increase the breathability of the border regions of the dressing, and so reduce the likelihood of maceration of the healthy peri-wound skin to which the dressing 100 is adhered. The perforations 135 may be formed during manufacture of the skin contact layer 130, in an analogous manner to the way that the perforations 125 are formed in the film 120. Some of the perforations 135 are also shown as hidden detail in Figure 1. If the backing layer 140 were of transparent material, the perforations 135 would be visible through it, across the full extent of the border region of the dressing 100.

The dressing may be manufactured by the following general method.

First, the MBPU/silicone gel composite used to form the skin contact layer 130 is produced in the manner described in WO2007/113597. In general terms, this involves applying silicone gel precursors to a sheet of melt-blown polyurethane (MBPU), the underside of which carries a coating of acrylic adhesive and a temporary protective backing, eg of plastics film or paper. Once the silicone gel precursors have cured, to produce a hydrophobic silicone gel, a temporary cover, again of plastics film or paper material, is applied to the gel. Perforations corresponding to the perforations 125 are then formed in the composite, and large openings that will constitute the central opening 133 of the skin contact layer 130 referred to above are cut out.

In a separate operation, a laminate of the foam that is used to form the pad 110 and the perforated film 120 is produced by bonding sheets of foam and perforated film material together, as described above. Individual pieces of the desired shape and size are then cut out to form the laminated pad 110 and film 120.

The pieces of laminated pad 110 and film 120 are then positioned on a sheet of breathable polyurethane film (which will form the backing layer 140 of the finished dressing 100). The temporary protective backing is removed from the underside of the MBPU to expose the acrylic adhesive and the MBPU/silicone gel composite is applied to the polyurethane film with the large openings in registration with the pieces of laminated pad 110 and film 120. Finally, the temporary protective cover is removed from the silicone gel and replaced with appropriately formed release liners 150a-c, and individual dressings 100 are punched out and sterile-packaged. Sterilisation may be carried out by any suitable means, eg with the use of gamma irradiation or ethylene oxide.

In use, a dressing 100 is removed from its packaging and the release liners 150a-c are peeled off. The dressing 100 is then applied to a wound, with the exposed film 120 overlying the wound and the surrounding silicone gel of the skin contact layer 130 being attached to the peri-wound skin. Exudate from the wound is able to pass through the perforations 125 and is absorbed by the foam pad 110. At the same time, however, contact between wound exudate and the film 120 causes the film to gradually dissolve. As it does so, the antimicrobial components of the dressing 100 (eg manuka honey, manuka oil and PHMB) are released into the wound site.

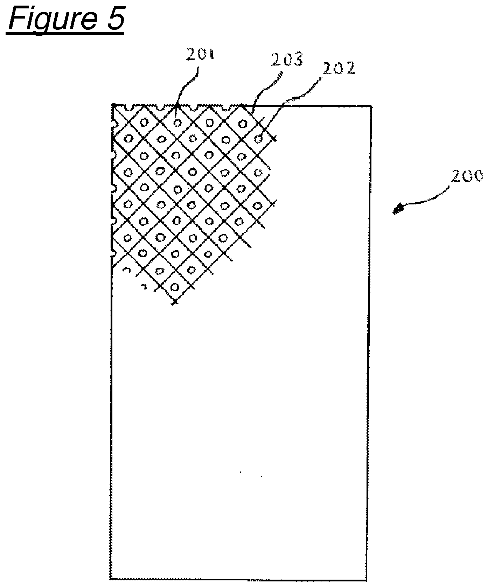

Figure 5 shows a second embodiment of a wound dressing according to the invention, designated 200.

The dressing 200 is similar to the laminate described above in that it comprises an absorbent foam pad that is laminated to a honey-containing film 201. However, in this embodiment the perforations 202, shown on a portion of the dressing of Figure 5, pass through the whole dressing 200. The film 201 may be a dissolvable antimicrobial film prepared as described in the Example above but the perforations are not introduced until after the film 201 is laminated to the foam pad. The foam pad and unperforated film are first bonded together by applying heat and pressure, eg by passing the layered foam and unperforated film through a pair of heated rollers. A regular array of perforations 202 is subsequently introduced into the laminate as a whole, ie through the film 201 and the foam, by compressing the laminate between a sonotrode and a substrate and applying high frequency mechanical vibrations. Apparatus by which the perforations are introduced into the film is similar to that depicted schematically in Figure 4. The laminate is introduced into the apparatus 10 in place of the film/substrate 2.

As the perforations 202 in the dressing 200 are formed by compressing the absorbent foam pad and the film, the dressing remains compressed at the point at which the perforations are introduced. This results in a quilted appearance with the uncompressed areas of the foam pad forming peaks 203 between the perforations 202 in the laminate as illustrated by the lines 203 in Figure 5.

The dressing of Figure 5 may be applied directly to a wound or alternatively, pieces of the laminated product may be cut out and incorporated into a wound dressing, for instance in place of the laminate in the wound dressing shown in Figures 1 to 3.

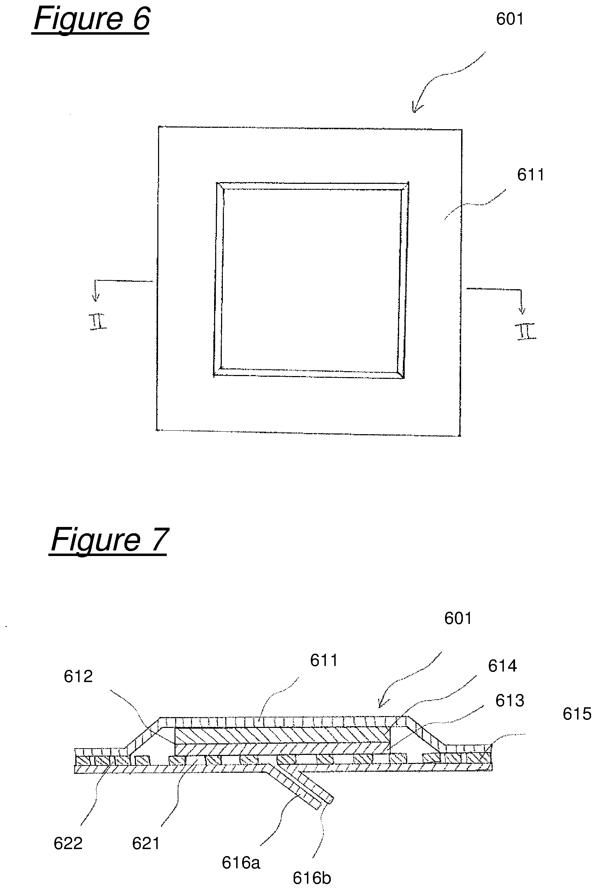

Referring now to Figures 6 and 7, there is shown a wound dressing, generally designated 601. The dressing 601 is generally square in form and comprises a backing layer of microporous, gas- and vapour-permeable polyurethane film 611, to a central portion of the underside of which is affixed an absorbent body 612 (see Figure 7).

The absorbent body 612 comprises a layer of polyurethane foam 613, the upper (as viewed in Figure 7) surface of which is bonded to a sheet of superabsorbent material 614. The sheet of superabsorbent material 614 comprises particles of sodium polyacrylate polymer encapsulated between two layers of tissue paper carrier material.

A perforated skin contact layer 615 extends across the whole extent of the underside of the dressing 601, and is covered by a protective release liner 616a,616b.

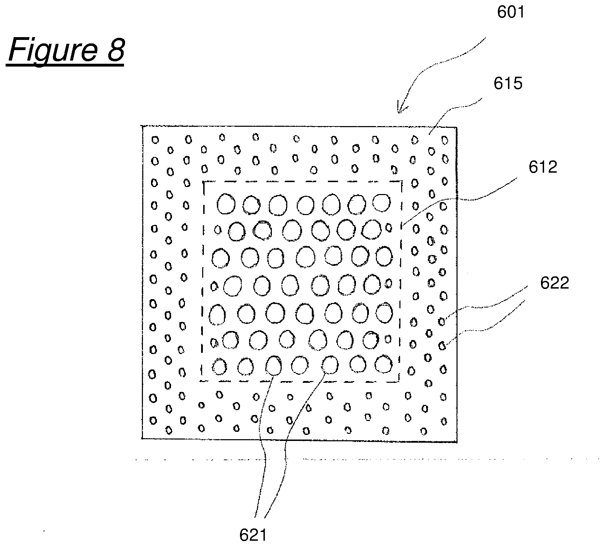

As can be seen in the underside plan view of Figure 8, the contact layer 615 is formed with two different arrays of perforations: larger perforations 621 and smaller perforations 622. The larger perforations 621 are formed in the central portion of the dressing 601 and coincide with the absorbent body 612. These perforations 621 have approximate diameter 5mm, to permit wound exudate to pass from the wound into the foam 613 and the superabsorbent material 614. The smaller perforations 622 are formed in the border of the contact layer 615, ie the portion surrounding, but not extending over, the absorbent body 612. These perforations have a diameter of 1-2mm and permit the passage of water vapour and gas from the surface of the skin surrounding a wound, through the backing layer 611. Maceration of the peri-wound skin is thereby avoided, but the smaller perforations 622 do not substantially impair secure retention of the dressing 601 on the skin. The silicone gel layer 615 contacts not only the healthy skin around the wound, but also the wound itself, with the absorbent body 612 exposed to the wound through the large perforations 621. The non-adherent properties of the silicone gel skin contact layer 615 permit the dressing 601 to be removed from the wound without trauma or pain.

The silicone gel skin contact layer 615 comprises a sheet of melt-blown polyurethane that carries a coating of silicone gel. The reverse side of the melt-blown polyurethane is coated with acrylic adhesive by which it is affixed to the border of the backing layer 611 and to the absorbent body 612.

The wound dressing is supplied with a two-part release liner 616a,616b (shown in Figure 7, but not in Figure 8), which is removed from the dressing 601 immediately prior to use.

When the dressing 601 is applied to a wound, wound exudate passes through the larger perforations 621 into the absorbent body 612, the superabsorbent material 614 in particular having a substantial absorptive capacity.

The dressing may be manufactured by the following general method.

First, a pre-laminate comprising the silicone gel layer is produced in the manner described in WO2007/113597. In general terms, this involves applying silicone gel precursors to a sheet of melt-blown polyurethane (MBPU), the underside of which carries a coating of acrylic adhesive and a temporary protective backing, eg of plastics film or paper. Once the silicone gel precursors have cured, to produce a hydrophobic silicone gel, a temporary cover, again of plastics film or paper material, is applied to the gel. Perforations corresponding to the larger and smaller perforations 621,622 are then formed in the pre-laminate.

In a separate operation, the absorbent body is produced by sandwiching sheets of foam and superabsorbent material together. Individual squares, or other appropriate shapes, are then cut out.

The squares of the absorbent body are then positioned on a sheet of breathable polyurethane film (which constitutes the backing layer 611 of the finished dressing 601). The temporary protective backing is removed from the underside of the MBPU to expose the acrylic adhesive and the pre-laminate is applied to the polyurethane film with the large perforations 621 in registration with the squares of absorbent body 612. Finally, the temporary protective cover is removed from the silicone gel and replaced with appropriately formed release liners 616a,616b, and individual dressings 601 are punched out and sterile-packaged.

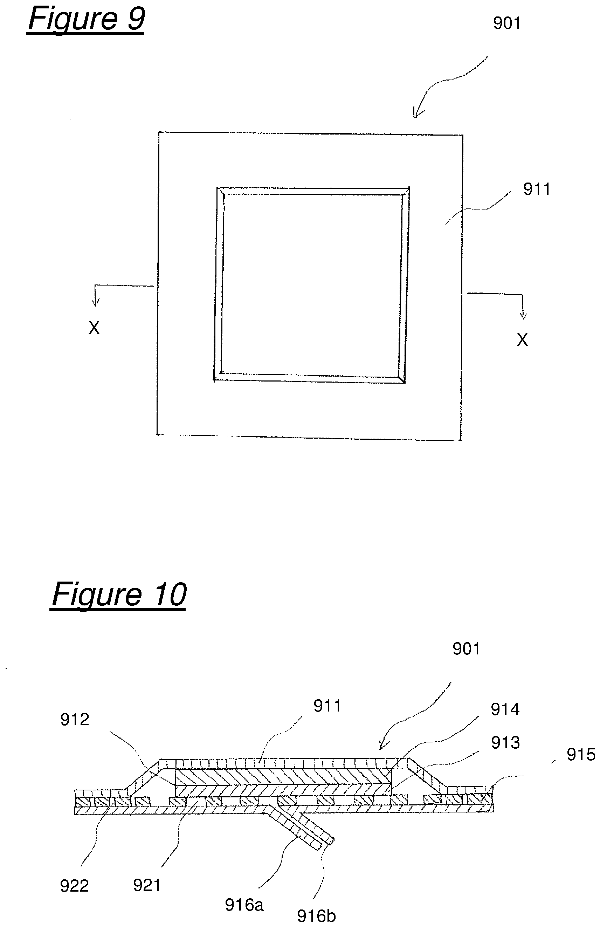

In Figures 9 and 10, there is shown an alternative wound dressing according to the invention, generally designated 901. The dressing is generally square in form and comprises a backing layer of microporous polyurethane film 911, to the underside of a central portion of which is affixed a square of an absorbent component 912 according to the invention (see Figure 10).

The absorbent component 912 comprises a wound-facing sheet of polyurethane foam 913, the outer surface of which is bonded to a sheet of superabsorbent material 914. The sheet of superabsorbent material 914 comprises particles of sodium polyacrylate polymer encapsulated between two layers of tissue paper carrier material.

A skin contact layer 915 extends across the whole extent of the underside of the dressing 901. The skin contact layer 915 comprises a sheet of melt-blown polyurethane that carries a coating of silicone gel. The reverse side of the melt-blown polyurethane is coated with acrylic adhesive by which it is affixed to the border of the backing layer 911 and to the absorbent component 912.

The central part of the skin contact layer 915 is formed with a first set of relatively large perforations 921, and the peripheral part with a second set of smaller perforations 922. The first set of perforations 921 extend across the central portion of the dressing and coincide with the absorbent component 912. These perforations are larger and have approximate diameter 5mm, to permit wound exudate to pass from the wound into the foam 913 and the superabsorbent material 914. The second set of perforations 922 extend across the border of the silicone gel contact layer 915, ie the portion surrounding, but not extending over, the absorbent component 912. These perforations 922 are smaller in diameter and result in the silicone gel layer 915 being breathable. The silicone gel layer 915 contacts not only the healthy skin around the wound, but also the wound itself, with the absorbent component 912 exposed to the wound through the large perforations 921. The non-adherent properties of the silicone gel skin contact layer 915 permit the dressing to be removed from the wound without trauma or pain.

The wound dressing 901 is supplied with a two-part release liner 916a,916b, which is removed from the dressing 901 immediately prior to use, in order to expose the absorbent component 912 through the large perforations 921.

When the dressing is applied to a wound, the foam layer 913 allows wound exudate to pass through it into the superabsorbent material 914 where it is absorbed.

The dressing may be manufactured by the following general method.

First, a pre-laminate comprising the silicone gel layer is produced in the manner described in WO2007/113597. In general terms, this involves applying silicone gel precursors to a sheet of melt-blown polyurethane (MBPU), the underside of which carries a coating of acrylic adhesive and a temporary protective backing, eg of plastics film or paper. Once the silicone gel precursors have cured, to produce a hydrophobic silicone gel, a temporary cover, again of plastics film or paper material, is applied to the gel. Perforations corresponding to the larger and smaller perforations 921,922 are then formed in the pre-laminate.

In a separate operation, the absorbent component 912 is produced by sandwiching sheets of foam and superabsorbent material together. Individual squares, or other appropriate shapes, are then cut out.

The squares of the absorbent component 912 are then positioned on a sheet of breathable polyurethane film (which constitutes the backing layer 911 of the finished dressing). The temporary protective backing is removed from the underside of the MBPU to expose the acrylic adhesive and the pre-laminate is applied to the polyurethane film with the large perforations in registration with the squares of absorbent component 912. Finally, the temporary protective cover is removed from the silicone gel and replaced with appropriately formed release liners, and individual dressings are punched out and sterile-packaged.

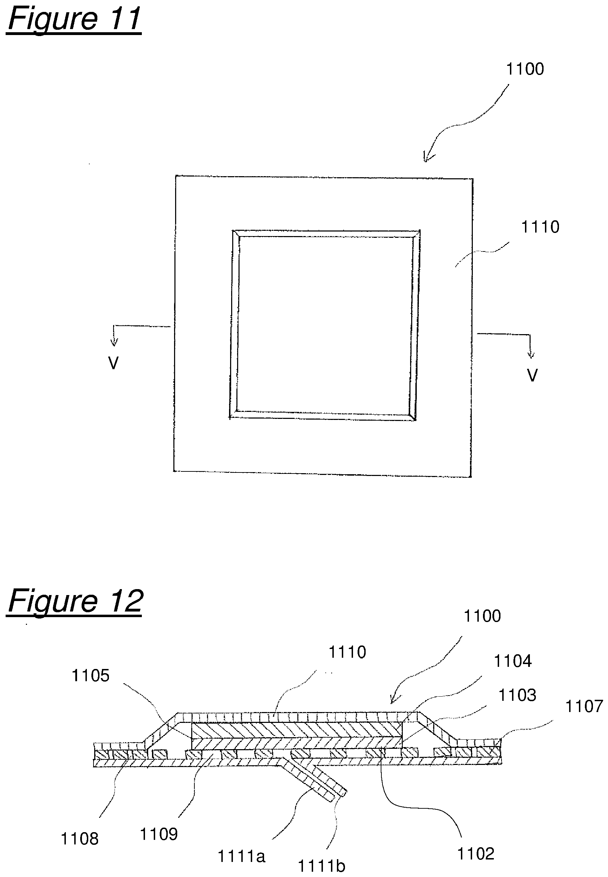

Turning now to Figures 11 and 12, an alternative embodiment of a wound dressing according to the invention is generally designated 1100. The dressing 1100 is generally square in shape, but it will be understood that the shape and dimensions of the dressing may vary widely, depending for instance on the size and nature of the wound to be dressed and the part of the body to which the dressing is to be applied. The dressing comprises a backing layer of microporous, gas- and vapour-permeable polyurethane film 1110, to a central portion of the underside of which is affixed an absorbent body 1105 (see Figure 12).

Figure 11 is a plan view of the dressing 1100 from above, ie from the non-wound-facing side. The structure of the dressing 1100 is more evident from the cross-sectional view of Figure 12 (in which the thickness of the various components is depicted on an exaggerated scale).

As can be seen most clearly in Figure 12, the dressing 1100 comprises an absorbent body 1105 that is laminated at its underside (as viewed in Figure 12) to a perforated honey-containing film 1102. The film 1102 may be a dissolvable antimicrobial film prepared as previously described. The absorbent body 1105 and film 1102 are of identical length and width, and have been bonded together by means of a fusible web, as previously described.

The absorbent body 1105 comprises a layer of polyurethane foam 1103, the upper (as viewed in Figure 12) surface of which is bonded to a sheet of superabsorbent material 1104. The sheet of superabsorbent material 1104 comprises a fibrous, non-woven mat formed of superabsorbent fibres.

A perforated skin contact layer 1107 extends across the whole extent of the underside of the dressing 1100, and is covered by a protective release liner 1111a 1111b.

The contact layer 1107 is formed with two different arrays of perforations: larger perforations 1109 and smaller perforations 1108. The larger perforations 1109 are formed in the central portion of the dressing 1100 and coincide with the absorbent body 1105. These perforations 1109 have approximate diameter 5mm, to permit wound exudate to pass from the wound into the foam 1103 and the superabsorbent material 1104. The smaller perforations 1108 are formed in the border of the contact layer 1107, ie the portion surrounding, but not extending over, the absorbent body 1105. These perforations have a diameter of 1-2mm and permit the passage of water vapour and gas from the surface of the skin surrounding a wound, through the backing layer 1110. Maceration of the peri-wound skin is thereby avoided, but the smaller perforations 1108 do not substantially impair secure retention of the dressing 1100 on the skin. The silicone gel layer 1107 contacts not only the healthy skin around the wound, but also the wound itself, with the absorbent body 1105 exposed to the wound through the large perforations 1109. The non-adherent properties of the silicone gel skin contact layer 1107 permit the dressing 1100 to be removed from the wound without trauma or pain.

The silicone skin gel contact layer 1107 comprises a sheet of melt-blown polyurethane that carries a coating of silicone gel. The reverse side the melt-blown polyurethane is coated with acrylic adhesive by which it is affixed to the border of the backing layer 1110 and to the absorbent body 1105.

The wound dressing is supplied with a two-part release liner 1111a 1111b (see Figure 12), which is removed from the dressing 1100 immediately prior to use.

When the dressing 1100 is applied to a wound, wound exudate passes through the larger perforations 1108 into the absorbent body 1105, the superabsorbent material 1104 in particular having a substantial absorptive capacity.

## Claims

1. A wound dressing comprising an absorbent body that is laminated to, or is in intimate contact with, a perforated film comprising honey, wherein the film is formed with a regular array of perforations and, in use, is exposed to a wound to which the dressing is applied, such that exudate from the wound passes through perforations in the film and is absorbed by the absorbent body, and wherein the film dissolves upon exposure to wound exudate, thereby releasing the honey.

2. A wound dressing according to Claim 1, wherein the perforated film comprises a carrier material.

3. A wound dressing according to any preceding claim, wherein the carrier is a polymeric material.

4. A wound dressing according to Claim any preceding claim, wherein the perforated film has a thickness of from 200µm to 2mm.

5. A wound dressing according to Claim any preceding claim, wherein the honey content of the film is from about 25% to about 75% w/w.

6. A wound dressing according to any preceding claim, wherein the film is laminated to the absorbent body, and perforations extend through both the film and the absorbent body.

7. A wound dressing according to any preceding claim, wherein the dressing further comprises a backing layer with a moisture vapour transmission rate of at least 300g/m²/24h at 37°C and 100% to 10% relative humidity difference.

8. A method of manufacturing a perforated film for use in a dressing according to Claim 1, comprising the steps of:
a) producing a fluid precursor composition comprising a carrier material, one or more benefit agents, and a liquid medium; and
b) casting the precursor composition into a sheet;
c) causing or allowing the precursor composition to at least partially solidify; and
d) forming perforations in the at least partially solidified film.

9. The method according to Claim 8, wherein the precursor composition contains about 20% to about 80% w/w of a liquid medium.

10. The method according to Claim 8, wherein to cause the precursor composition to at least partially solidify the precursor composition in step c) is heated.

11. The method according to Claim 8, wherein the perforations are produced by contacting perforating elements with the film and subjecting the film to high frequency mechanical vibrations.

## Patentansprüche

1. Wundverband, umfassend einen absorbierenden Körper, der auf einen perforierten Film umfassend Honig laminiert ist oder damit in engem Kontakt ist, wobei der Film mit einer regelmäßigen Anordnung von Perforationen gebildet ist und in Verwendung einer Wunde ausgesetzt ist, auf die der Verband angewendet wird, sodass Exsudat aus der Wunde durch die Perforationen in dem Film verläuft und von dem absorbierenden Körper absorbiert wird, und wobei sich der Film bei Aussetzung an das Wundexsudat ausgesetzt ist, wodurch der Honig freigesetzt wird.

2. Wundverband nach Anspruch 1, wobei der perforierte Film ein Trägermaterial umfasst.

3. Wundverband nach einem der vorherigen Ansprüche, wobei der Träger ein polymeres Material ist.

4. Wundverband nach Anspruch eines vorherigen Anspruchs, wobei der perforierte Film eine Stärke von 200 µm bis 2 mm aufweist.

5. Wundverband nach Anspruch eines vorherigen Anspruchs, wobei der Honiggehalt des Films etwa 25 % bis etwa 75 % Gew./Gew. ist.

6. Wundverband nach einem der vorherigen Ansprüche, wobei der Film auf den absorbierenden Körper laminiert ist und Perforationen sich sowohl durch den Film als auch durch den absorbierenden Körper erstrecken.

7. Wundverband nach einem der vorherigen Ansprüche, wobei der Verband ferner eine Trägerschicht mit einer Wasserdampfdurchlässigkeitsrate von mindestens 300 g/m²/24Std. bei 37 °C und 100 % bis 10 % relativer Feuchtigkeitsdifferenz umfasst.

8. Verfahren zum Fertigen eines perforierten Films zur Verwendung in einem Verband nach Anspruch 1, umfassend die folgenden Schritte:
a) Herstellen einer fluiden Vorläuferzusammensetzung, umfassend ein Trägermaterial, ein oder mehrere vorteilhafte Mittel und ein flüssiges Medium; und
b) Gießen der Vorläuferzusammensetzung in eine Platte;
c) Bewirken oder Zulassen, dass die Vorläuferzusammensetzung mindestens teilweise erstarrt; und
d) Bilden von Perforationen in dem mindestens teilweise verfestigten Film.

9. Verfahren nach Anspruch 8, wobei die Vorläuferzusammensetzung etwa 20 bis etwa 80 % Gew./Gew. eines flüssigen Mediums enthält.

10. Verfahren nach Anspruch 8, wobei die Vorläuferzusammensetzung, um die Vorläuferzusammensetzung zu veranlassen, mindestens teilweise zu erstarren, in Schritt c) erhitzt wird.

11. Verfahren nach Anspruch 8, wobei die Perforationen erzeugt werden, indem perforierende Elemente mit dem Film in Kontakt gebracht werden und der Film hochfrequenten mechanischen Schwingungen ausgesetzt wird.

## Revendications

1. Pansement pour plaie comprenant un corps absorbant qui est stratifié sur, ou est en contact intime avec, un film perforé comprenant du miel, ledit film étant formé d'une matrice régulière de perforations et, lors de l'utilisation, étant exposé à une plaie sur laquelle le pansement est appliqué, de sorte que l'exsudat provenant de la plaie passe à travers les perforations dans le film et soit absorbé par le corps absorbant, et ledit film se dissolvant lors de l'exposition à l'exsudat de la plaie, ce qui permet de libérer le miel.

2. Pansement pour plaie selon la revendication 1, ledit film perforé comprenant un matériau support.

3. Pansement pour plaie selon l'une quelconque des revendications précédentes, ledit support étant un matériau polymère.

4. Pansement pour plaie selon une revendication l'une quelconque des revendications précédentes, ledit film perforé ayant une épaisseur allant de 200 µm à 2 mm.

5. Pansement pour plaie selon une revendication l'une quelconque des revendications précédentes, la teneur en miel du film allant d'environ 25 % à environ 75 % en p/p.

6. Pansement pour plaie selon l'une quelconque des revendications précédentes, ledit film étant stratifié sur le corps absorbant et lesdites perforations s'étendant à travers à la fois le film et le corps absorbant.

7. Pansement pour plaie selon l'une quelconque des revendications précédentes, ledit pansement comprenant en outre une couche de renforcement présentant une vitesse de transmission de la vapeur humide d'au moins 300 g/m²/24h à 37°C et une différence d'humidité relative de 100 % à 10 %.

8. Procédé de fabrication d'un film perforé destiné à être utilisé dans un pansement selon la revendication 1, comprenant les étapes de :
a) production d'une composition précurseur fluide comprenant un matériau support, un ou plusieurs agents bénéfiques, et un milieu liquide ; et
b) moulage de la composition précurseur en feuille ;
c) provocation ou permission de la solidification au moins partielle de la composition précurseur ; et
d) formation de perforations dans le film au moins partiellement solidifié.

9. Procédé selon la revendication 8, ladite composition précurseur contenant environ 20 % à environ 80 % en p/p d'un milieu liquide.

10. Procédé selon la revendication 8, pour la provocation de la solidification au moins partielle de la composition précurseur ladite composition précurseur à l'étape c) étant chauffée.

11. Procédé selon la revendication 8, lesdites perforations étant produites par mise en contact d'éléments perforants avec le film et soumission du film à des vibrations mécaniques de haute fréquence.
